Europäisches Patentamt

European Patent Office

Office européen des brevets

(19)

(11) Veröffentlichungsnummer : **0 017 115**
**B1**

# EUROPÄISCHE PATENTSCHRIFT

(12)

(45) Veröffentlichungstag der Patentschrift :
16.02.83

(51) Int. Cl.³ : **C 12 N 11/14**

(21) Anmeldenummer : **80101526.4**

(22) Anmeldetag : **22.03.80**

(54) **Verfahren zur Herstellung von in Kieselgel eingebetteten enzymatisch aktiven Präparaten.**

(30) Priorität : 26.03.79 DE 2911776

(43) Veröffentlichungstag der Anmeldung :
15.10.80 Patentblatt 80/21

(45) Bekanntmachung des Hinweises auf die Patenterteilung : 16.02.83 Patentblatt 83/07

(84) Benannte Vertragsstaaten :
**AT BE CH DE FR GB IT LU NL SE**

(56) Entgegenhaltungen :
**Keine**

(73) Patentinhaber : BASF Aktiengesellschaft
Carl-Bosch-Strasse 38
D-6700 Ludwigshafen (DE)

(72) Erfinder : Tschang, Chung-Ji, Dr.
Albrecht-Duerer-Ring 14
D-6710 Frankenthal (DE)
Erfinder : Klefenz, Heinrich, Dr.
Schillerstrasse 10
D-6701 Hochdorf-Assenheim 2 (DE)
Erfinder : Sanner, Axel, Dr.
Lorschner Ring 2C
D-6710 Frankenthal (DE)
Erfinder : Zahn, Wolfgang, Dr.
Parkstrasse 6
D-6701 Altrip (DE)

EP 0 017 115 B1

Jouve, 18, rue St-Denis, 75001 Paris, France

Verfahren zur Herstellung von in Kieselgel eingebetteten enzymatisch aktiven Präparaten

Die Erfindung betrifft ein Verfahren zur Herstellung von in Kieselgel eingebetteten enzymatisch aktiven Präparaten.

Unlösliche Präparate mit enzymatischer Aktivität finden u. a. Anwendung in der medizinischen Analytik sowie bei der Herstellung von Grundstoffen für die Nahrungsmittel- und Arzneimittelindustrie. Weiter eignen sie sich zur Synthese optisch aktiver Substanzen. Die bisherigen Präparate dieser Art zeigen gegenüber gelösten oder suspendierten enzymatisch aktiven Präparaten einige Vorteile hinsichtlich der leichten Abtrennbarkeit vom Substrat, der Stabilität, der Nicht-Verunreinigung der Reaktionsprodukte sowie der Möglichkeit, Reaktionen kontinuierlich durchzuführen. Sie sind jedoch nicht frei von erheblichen Mängeln. So befriedigen die adsorptive und die ionische Bindung der enzymatisch aktiven Präparate an Trägermaterialien im allgemeinen nicht die Ansprüche, die hinsichtlich der Festigkeit und Dauerhaftigkeit an sie gestellt werden. Weiter müssen bei diesen Verfahren enzymatisch aktive Präparate in Form von reinen oder weitgehend vorgereinigten Enzymen verwendet werden. Dies gilt auch dann, wenn eine kovalente Bindung im Präparat vorliegt, sofern es sich nicht um eine Vernetzung handelt, die ihrerseits auf Zellmaterial und Mikroorganismen oder Bruchstücke davon beschränkt ist.

Ein Verfahren, welches diese Nachteile weitgehend vermeidet, ist in der DE-A 1 939 347 angegeben, in der die Herstellung eines wäßrigen Siliciumdioxidgels in Gegenwart einer enzymatisch aktiven Substanz beschrieben ist. Bei diesem Verfahren fällt eine gallertartige Masse an, die durch aufwendige und verlustreiche Maßnahmen in eine für die Beschickung von Reaktoren geeignete Form gebracht werden muß. Darüber hinaus geht man bei der Herstellung des Gels von einem Siliciumdioxidsol aus, welches durch ein zeitraubendes und umständliches Verfahren gewonnen wird.

Es wurde nun ein einfaches Verfahren gefunden, welches diese Nachteile vermeidet.

Gegenstand der Erfindung ist ein Verfahren zur Herstellung von in Kieselgelen eingebetteten enzymatisch aktiven Präparaten, welches darin besteht, daß man wäßrige Mischungen von enzymatisch. aktiven Präparaten und gelösten Alkali und/oder Ammoniumsilikaten in organischen, mit Wasser nicht mischbaren Flüssigkeiten suspendiert und anschließend in ein wasserunlösliches Gel überführt.

Unter enzymatisch aktiven Präparaten sind vorzugsweise an Teilchen gebundene Enzyme, Zellfragmente und -fraktionen, getrocknete Zellen, Drüsensekrete, Mikroorganismen sowie Sporen von Pilzen und Mikroorganismen zu verstehen. Es können auch Enzyme in gelöstem suspendiertem, dispergiertem oder trockenem Zustand eingesetzt werden.

Beispiele hierfür sind insbesondere : Mikroorganismen der Gattung Streptomyces, Arthrobacter und Bacillus mit Glucoseisomerase-Aktivität, Escherichia coli mit Penicillinacylase-Aktivität, Arthrobacter simplex zur Dehydrierung von Steroiden, Saccharomyces cerevisiae zur Reduzierung von Ketonen, Curvularia lunata zur Hydroxilierung von Steroiden und Aspergillus ochraceus mit Aminoacylase-Aktivität. Ferner eignen sich Trypsin, Chymotrypsin, Pankreatin, α- und β-Amylase, Ribonucleasen, Desoxyribonucleasen, Cellulase, Maltase, Pectinase, Chitinase, Pepsin, Bromelain, Keratinase, Amyloglycosidase, Lipase, Cholinesterase, Lecithinase, Phosphatase, Alginase, Asparaginase, Glutaminase, Urease, Lactase, Penicillinamidase, Penicillinase, Glucoseisomerase, Glucoseoxidase, Katalase, Peroxidase, Lipoxidase, Xanthinoxidase, Cytochromreductase, Milchsäureoxidase, Aminosäureoxidase, Rennin, Ficin, Subtilisin, Tannase, Phenoloxidase, Pullulanase, Pankreatin, Isoamylase, Hexokinase, Galactoseoxidase, Diaphorase, Aldolase, Glykolsäureoxidase, Luciferase, Aldehydoxidase, Naringinase, Uricase, Glutathionreductase, Nitritoreductase, Nitratreductase, Bernsteinsäuredehydrogenase, Catecholoxidase, β-Fructosidase, Aminosäureacylase und Urokinase bzw. Präparationen mit diesen Enzymen.

Als silikatische Lösungen kommen die Lösungen von Natrium-, Kalium- oder Ammoniumsilikaten mit einer Konzentration von 8 bis 27 Gewichtsprozent, vorzugsweise 11 bis 24 Gewichtsprozent, bezogen auf $SiO_2$, in Betracht.

Als mit Wasser nicht mischbare Lösungsmittel werden vorzugsweise solche auf der Basis von aliphatischen, cycloaliphatischen und aromatischen Kohlenwasserstoffen oder halogenierte Kohlenwasserstoffe, wie 1.1.1-Trichlorethan verwendet.

Es ist zweckmäßig, den Suspensionsvorgang durch Suspensionshilfsmittel zu unterstützen. Hierfür sind unter anderem die Substanzen geeignet, welche bei der Durchführung von Wasser-in-Öl-Suspensionspolymerisationen (umgekehrte Suspensionspolymerisationen) verwendet werden. Beispiele hierfür sind Sorbitanester sowie Substanzen gemäß US-A 2 982 749, DE-A 2 009 218, DE-A 2 634 436 und DE-A 2 10 372.

Die Gelierung der Silicate erfolgt in bekannter Weise durch Zugabe von Elektrolyten oder organischen, mit Wasser mischbaren Lösungsmitteln oder vorzugsweise durch Herabsetzen des pH-Wertes durch Zugabe eines in Wasser und in der organischen Flüssigkeit löslichen Agens.

Besonders gut gelingt die Gelierung, wenn man zu der Suspension, welche aus der organischen Flüssigkeit und dem wäßrigen, die Silikate und das enzymatisch aktive Präparat enthaltenden Gemisch besteht, eine in beiden Phasen lösliche organische Säure, vorzugsweise Essigsäure, zutropft, wobei Neutralisation und Fällung eintritt. Um eine gleichmäßige Fällung zu erzielen, kann die Säure mit einer geringen Menge der organischen Flüssigkeit verdünnt werden. Durch die Wahl der Menge an Säure oder eines Säurederivats, z. B. eines Anhydrids oder Halogenids, kann der pH-Wert am Ende der Fällung auf das immobilisierte Enzympräparat abgestimmt werden.

Überraschenderweise bleibt bei diesem Verfahren trotz der Alkali-Belastung, welche für einige Sekunden bis mehrere Minuten auftritt, die enzymatische Aktivität weitgehend erhalten, sofern es sich nicht um ein stark alkaliempfindliches Enzym handelt. Durch Zugabe einer kleinen Menge Säure zu der Alkali- oder Ammoniumsilikatlösung vor dem Vermischen mit der enzymatisch aktiven Substanz kann diese Belastung verringert werden.

Es empfiehlt sich, bei der Gelierung durch Einarbeiten von inerten organischen oder anorganischen Stoffen zusammen mit dem enzymatisch aktiven Präparat die Quellung der entstehenden Präparate in Wasser zu verringern. Auch die Dichte kann so variiert werden. Geeignete inerte Stoffe sind z. B. Bimssteinpulver, Glaspulver oder -fasern, Kieselgur, Zeolithe, Pulver von organischen Polymeren, Aktivkohle oder schwerlösliche Salze.

Eine zusätzliche Verfestigung kann erzielt werden, wenn man zu der wäßrigen silikatischen Mischung lösliche Sulfate oder Phosphate gibt und nach der Gelierung das erhaltene Präparat mit einer wäßrigen Lösung von Salzen behandelt, deren Kationen schwerlösliche Sulfate bzw. Phosphate bilden. Beispiele für solche Salze sind Calciumchlorid und Bariumchlorid.

Die nach dem neuen Verfahren hergestellten Enzympräparationen lassen sich sehr einfach anwenden und sind, auch bei häufiger Benutzung, sehr lange Zeit stabil.

Die folgenden Beispiele erläutern die Erfindung. Die darin erwähnten Mikroorganismen dienen lediglich als Proben für enzymatisch aktive Substanzen. Sollten die Mikroorganismen irgendwann aus irgendwelchen Gründen nicht mehr zur Verfügung stehen, können sie durch beliebige andere Mikroorganismen ersetzt werden, die enzymatische Aktivität besitzen.

## Beispiel 1

### Immobilisierung von Backhefe

Apparatur : 500 ml-Dreihalskolben mit Rührer und Tropftrichter
Es werden folgende Mischungen bzw. Lösungen hergestellt :

1) 0,04 g Suspensionshilfsmittel (Schutzkolloid A gemäß DE-A 2 710 372) in 300 ml Toluol,
2) 4,7 g Calciumchlorid in 10 ml Wasser,
3) 7 g Trockenbackhefe und 12 ml Wasser,
4) 20 g Natriumsilikatlösung (26 %ig, bezogen auf $SiO_2$) mit 12 ml Wasser und 12 ml 1N Schwefelsäure.

1) wird im Kolben vorgelegt. 4) wird rasch mit 3) vermischt und unter Rühren zu 1) gegeben, wobei es sich tröpfchenförmig verteilt. Innerhalb von 1 min wird 2) zugetropft, wobei sich das Hefe-Silikat-Gemisch verfestigt. Nach 1 min Rühren wird das Produkt auf einer groben Glasfritte abgesaugt, mehrfach mit Wasser gewaschen und schließlich bei 70 °C im Vakuum getrocknet.
Ausbeute : 13 g annähernd perlförmiges Produkt, Teilchengröße < 1 mm.

## Beispiel 2

### Immobilisierung von Backhefe

Apparatur und Ausführung wie Beispiel 1. In Abweichung von diesem bestand die Mischung 1) aus 0,04 g Suspensionshilfsmittel (Schutzkolloid A gemäß DE-OS 2 634 486), 150 ml Cyclohexan und 150 ml n-Octan und die Mischung 2) aus 4 ml konz. Salzsäure.
Ausbeute : 7 g sehr feinkörniges perlähnliches Produkt.

## Beispiel 3

### Immobilisierung von Backhefe

Apparatur und Ausführung wie Beispiel 2. In Abweichung von diesem bestand die Mischung 2) aus 12,1 g Acetanhydrid.
Ausbeute : 14 g perlförmiges Produkt.

## Beispiel 4

### Immobilisierung von Backhefe

Apparatur : 1 l-Dreihalskolben mit Rührer und Tropftrichter.
Es wurden die folgenden Mischungen bzw. Lösungen hergestellt :

1) 0,1 g Suspensionshilfsmittel (Schutzkolloid A gemäß DE-A 2 634 486) in 650 ml Cyclohexan,
2) 3 g Eisessig in 25 ml Cyclohexan,
3) 17,5 g Trockenbackhefe und 35 ml Wasser,
4) 50 g Natriumsilikatlösung (26 %ig, bezogen auf $SiO_2$), mit 12,5 ml Wasser verdünnt.

Die Ausführung erfolgte wie in Beispiel 1 beschrieben. In Abweichung von diesem wurde jedoch die Lösung 2) während 5 min zugetropft.
Ausbeute : 23 g annähernd perlförmiges Produkt.

Beispiel 5

Immobilisierung von Backhefe

Apparatur : wie Beispiel 4
Es wurden folgende Mischungen bzw. Lösungen verwendet :

1) 0,1 g Suspensionshilfsmittel (Schutzkolloid A gemäß DE-A 2 634 486), 300 ml Cyclohexan und 300 ml n-Octan,
2) 5 g Eisessig in 25 ml Cyclohexan,
3) 17,5 g Trockenbackhefe, 10 g Bimsstein-Pulver und 40 ml Wasser,
4) 50 g Natriumsilikatlösung (26 %ig, bezogen auf $SiO_2$), 30 ml Wasser und 30 ml 1 N Schwefelsäure.

Ausführung :
Die Fällung erfolgt wie in Beispiel 1. Das Produkt wird nach dem Absaugen des Cyclohexan/n-Octan-Gemisches 30 sec in 5 %iger Calciumchloridlösung gerührt, durch Zugabe von 1,25 g Eisessig vollends neutralisiert und dann noch 1 min weitergerührt. Nachdem zweimal 2 min in Wasser gerührt worden ist, wird das Produkt bei 50 °C in Vakuum getrocknet.
Ausbeute : 37,6 g annähernd perlförmiges Produkt.

Beispiel 6

Immobilisierung von Backhefe

Apparatur : wie Beispiel 4
Es wurden folgende Mischungen bzw. Lösungen verwendet :

1) 0,1 g Suspensionshilfsmittel (Schutzkolloid A aus DE-A 2 634 486), 325 ml Cyclohexan und 325 ml n-Octan,
2) 5 g Eisessig in 25 ml Cyclohexan,
3) 17,5 g Trockenbackhefe und 30 ml Wasser,
4) 50 g Natriumsilikatlösung (26 %ig, bezogen auf $SiO_2$), 10 ml Wasser, 20 ml 4,2 %ige Aluminiumsulfatlösung in 1 N Natronlauge und 25 ml 1 N Schwefelsäure.
Die Ausführung erfolgte wie in Beispiel 5 beschrieben.
Ausbeute : 31 g perlförmiges Produkt.

Beispiel 7

Immobilisierung von Backhefe

Apparatur : wie Beispiel 4
Es wurden folgende Mischungen bzw. Lösungen eingesetzt :

1) 0,1 g Suspensionshilfsmittel (Schutzkolloid A gemäß DE-A 2 634 486), 300 ml 1.1.1-Trichlorethan,
2) 60 ml Ethanol,
3) 31 g Trockenbackhefe und 72 ml Wasser,
4) 10 g Natriumsilikatlösung (26 %ig, bezogen auf $SiO_2$), 5,6 ml Wasser, 4,4 ml 4,2 %ige Aluminiumsulfatlösung in 1N Natronlauge und 5,6 ml 1N Schwefelsäure,
5) 13 ml 1N Salzsäure, 10 g Calciumchlorid und 500 ml Wasser.

Ausführung :
Die Fällung erfolgte wie in Beispiel 1. Nach der Fällung wird 5 min weitergerührt. Staubanteile wurden durch Dekantieren entfernt. Danach wird das gefällte Produkt 4 min in 5) gerührt, mit Wasser gewaschen und bei 50 °C in Vakuum getrocknet.
Ausbeute : 20 g feinkörniges Produkt.
Hefegehalt, durch Glühversuch ermittelt : 84 Gew.%.

Beispiel 8

Immobilisierung von Escherichia coli (mit Penicillinacylase-Aktivität).

Apparatur : wie Beispiel 4
Es wurden folgende Mischungen bzw. Lösungen hergestellt :

1)   0,065 g Suspensionshilfsmittel (Schutzkolloid A aus DE-A 2 634 486), 300 ml Cyclohexan und 300 ml n-Octan.
2)   3,2   g Eisessig in 15 ml Cyclohexan
3)   70    g wäßrige Suspension von Escherichia coli ATCC 11105. Gehalt an Trockenmasse : 15,8 Gew.%
4)   32,1   g Natriumsilikatlösung (26 %ig, bezogen auf SiO$_2$), 6,5 ml Wasser, 12,8 ml 4,2 %ige Aluminiumsulfatlösung in 1N Natronlauge und 16 ml 1N Schwefelsäure
5) 350    ml wäßrige Calciumchloridlösung, 5 %ig.

Ausführung :
Die Fällung erfolgte wie in Beispiel 1. Nach dem Absaugen wird das Produkt 1 min in 5) gerührt, danach erst mit 1 %iger, dann mit 0,5 %iger Natriumchloridlösung gewaschen und schließlich bei 50 °C in Vakuum getrocknet.
Ausbeute : 25 g perlförmiges Produkt.


Beispiel 9

Immobilisierung von Streptomyces wedmorensis (mit Glucose-isomerase-Aktivität)

Apparatur : wie Beispiel 4
Es wurden folgende Mischungen bzw. Lösungen hergestellt :

1) 0,065 g Suspensionshilfsmittel (Schutzkolloid A aus DE-A 2 634 486), 300 ml Cyclohexan und 300 ml 1.1.1-Trichlorethan,
2) 4  ▪ g Eisessig in 15 ml Cyclohexan,
3) 75    g wäßrige Suspension von Streptomyces wedmorensis ATCC 21175. Gehalt an Trockenmasse : 15,8 Gew.%,
4) 32,1   g Natriumsilikatlösung (26 %ig, bezogen auf SiO$_2$), 6,5 ml Wasser, 12,8 ml 4,2 %ige Natriumsulfatlösung in 1N Natronlauge und 16 ml 1N Schwefelsäure.

Ausführung :
Die Fällung erfolgte wie in Beispiel 1. Nach dem Absaugen wird das Produkt zweimal je 2 min in 500 ml 0,5 %iger Natriumchloridlösung gewaschen und dann bei 50 °C im Vakuum getrocknet.
Ausbeute : 20 g perlförmiges Produkt.

Beispiel 10

Immobilisierung von Arthrobacter species NRRL 3726 (mit Glucoseisomerase-Aktivität)

Apparatur : wie in Beispiel 4
Es wurden die Mischungen wie in Beispiel 9 verwendet, jedoch wurden als Mischung 3) 50 g wäßrige Suspension von Arthrobacter species NRRL 3726, Gehalt an Trockenmasse : 25,5 Gew.%, benutzt.
Die Ausführung erfolgte wie in Beispiel 9.
Ausbeute : 22,7 g perlförmiges Produkt.

Beispiel 11

Immobilisierung von β-Fructosidase

Apparatur : wie in Beispiel 1
Es wurden folgende Mischungen bzw. Lösungen hergestellt :

1) 0,02 g Suspensionshilfsmittel (Schutzkolloid A aus DE-A 2 634 486) in 150 ml 1.1.1-Trichlorethan, ·
2) 0,25 g Eisessig in 5 ml 1.1.1-Trichlorethan,
3) 50    mg β-Fructosidase in 5 ml 0,2M Natriumacetatpuffer, pH 4,65,
4) 10    g Natriumsilikatlösung (26 %ig, bezogen auf SiO$_2$), 10 ml Wasser, 4 ml 4,2 %ige Aluminiumsulfatlösung in 1N Natronlauge und 5 ml 1N Schwefelsäure.

Ausführung :
Die Fällung erfolgte wie in Beispiel 1. Nach dem Absaugen wird das Produkt mit Wasser gewaschen und dann bei Raumtemperatur im Vakuum getrocknet.
Ausbeute : 3,9 g.

Beispiel 12

Bestimmung der enzymatischen Aktivitäten.

Zur Bestimmung der Invertase-Aktivität (bei Backhefe und β-Fructosidase) wurde 1 g Präparat bei Raumtemperatur 1h in der 40 %igen (Gew.%) Lösung von Saccharose in 0,05 M Natriumacetatpuffer (pH 4,65) geschüttelt. Der Umfang der Hydrolyse wurde polarimetrisch ermittelt.
Zur Bestimmung der Glucoseisomere-Aktivität wurde 1 g Präparat 1h bei 70 °C in der wäßrigen Lösung folgender Substanzen geschüttelt :

Glucose, 1M
Kaliumphosphatpuffer, 0,2M
$MgSO_4 \cdot 7H_2O$, 0,01M
Cobaltchlorid, 0,001M.

Danach wurde eine Probe dieser Lösung, welche entsprechend der Umsetzung bis zu 60 mg Fructose enthielt, zu 5 ml Resorcin-Reagenz (0,05 % Resorcin in 4N HCl) gegeben. Die Lösung wurde 7 min auf 80 °C erhitzt und dann während 5 min auf 5 °C abgekühlt. 15 bis 30 min nach dem Abkühlen wurde die Extinktion bei 490 nm gemessen. Der Umsetzungsgrad wurde durch Vergleich mit einer Eichkurve ermittelt.
Zur Bestimmung der Penicillinacylase-Aktivität wurde 1 g Präparat in 6 ml Lösung von Penicillin G (Natriumsalz, 35 mg/ml) in 0,05 M Kaliumphosphatpuffer (pH 7,5) bei 45 °C geschüttelt. Nach 0,30 und 60 min wurde jeweils eine Probe von 0,1 ml entnommen und deren Gehalt an 6-Aminopenicillansäure bestimmt. Das Prinzip dieser Bestimmung ist die Bildung einer Schiff'schen Base aus 6-Aminopenicillansäure und p-Dimethylaminobenzaldehyd. Diese hat ein Absorptionsmaximum bei 409 nm. Die 6-Aminopenicillansäure enthaltende Probe (0,1 ml) wurde mit 4 ml 20 prozentiger Essigsäure und 2 ml 0,05 M NaOH vermischt und anschließend, um jegliche Trübung auszuschließen, 10 min zentrifugiert. 3,5 ml der zentrifugierten Lösung wurden mit 1 ml 0,5 %iger methanolischer p-Dimethylaminobenzaldehyd-Lösung versetzt und gründlich durchmischt. Nach 15 min wurde die Extinktion bei 409 nm gegen eine nicht 6-Aminopenicillansäure-haltige Probe gemessen. Der Hydrolysegrad wurde einer Eichkurve entnommen.
Die Ermittlung der Aktivitätsausbeute erfolgte durch Vergleich mit den Ergebnissen, welche unter gleichen Bedingungen bei Verwendung der nicht immobilisierten enzymatisch aktiven Substanz erhalten wurden.
Bei den Aktivitäts-Untersuchungen in der Säule wurde in Abweichung von den Untersuchungen im Batch bei der Verfolgung der Penicillinacylase-Aktivität 0,75 M Kaliumphosphatpuffer (pH 7,5) verwendet. Bei der Untersuchung der Glucoseisomerase-Aktivität wurde ohne Puffer bei pH 8,25 und mit einem Gehalt an Magnesiumchlorid von 0,004 Mol/l gearbeitet.
Der Gehalt an enzymatisch aktiver Substanz (bei der Immobilisierung von Zellmaterial bzw. Mikroorganismen) wurde — basierend auf Erfahrungen aus Glühversuchen — nach der folgenden empirischen Formel errechnet :

$$G = T/A \cdot 100 - 3$$

Darin ist G der Gehalt an enzymatisch aktiver Substanz im Produkt (in Gewichtsprozent), T die eingesetzte Trockenmasse an enzymatisch aktiver Substanz und A das Gewicht des nach den Beispielen erhaltenen Produkts (Ausbeute).
Die Ergebnisse der Aktivitätsbestimmungen sind der nachfolgenden Tabelle zu entnehmen.

Tabelle : Enzymatische Aktivität der immobilisierten Substanzen

| Präparat des Beispiels Nr. | Zellgehalt des Präparats (G) Gew.% | Substrat | Substrat-Konz. mg/ml Lsg. | Säulentest Durchflußrate $\left[\dfrac{\text{Säulenvol.}}{h}\right]$ | Umsatz % | Restaktivität % | Batch-Test Umsatz $\left[\dfrac{\text{mg Substrat}}{\text{g Zellpräparat. h}}\right]$ |
|---|---|---|---|---|---|---|---|
| 4 | 65 | Saccharose | 400 | 2 | 65 | — | — |
| 5 | 39 | Saccharose | 400 | 2 | 70 | 75 | 2 200 |
| 6 | 48 | Saccharose | 400 | 2 | 75-80 | 77 | 2 850 |
| 7 | 84 | Saccharose | — | — | — | 13,4 | 1 500 |
| 8 | 41 | Penicillin C | 35 | 0,25 | 60 | 8,9 | 342 |
| 9 | 50 | Glucose | 400 | 0,2 | 25 | 83 | 400 |
| 10 | 53 | Glucose | — | — | — | 70 | 360 |
| 11 | — | Saccharose | — | — | — | nicht bestimmbar | 250 |

## Ansprüche

1. Verfahren zur Herstellung von in Kieselgel eingebetteten enzymatisch aktiven Präparaten, dadurch gekennzeichnet, daß man wäßrige Mischungen von enzymatisch aktiven Präparaten und gelösten Alkali- und/oder Ammoniumsilikaten in organischen, mit Wasser nicht mischbaren Flüssigkeiten suspendiert und anschließend in ein wasserunlösliches Gel überführt.

2. Verfahren nach Anspruch 1, dadurch gekennzeichnet, daß als enzymatisch aktive Präparate aktive Zellen oder Zellbruchstücke mikrobiologischen, pflanzlichen, tierischen oder humanen Ursprungs verwendet werden.

3. Verfahren nach Anspruch 1, dadurch gekennzeichnet, daß die Bildung einer Suspension durch ein Suspensionshilfsmittel erleichtert wird.

4. Verfahren nach Anspruch 1 bis 3, dadurch gekennzeichnet, daß die Gelierung durch Herabsetzen des pH-Wertes mittels eines in Wasser und in der organischen, mit Wasser nicht mischbaren Flüssigkeit löslichen Agens, vorzugsweise einer organischen Säure, erfolgt.

5. Verfahren nach Anspruch 1 bis 4, dadurch gekennzeichnet, daß die Gelierung in Gegenwart eines inerten Stoffes erfolgt.

6. Verfahren nach Anspruch 1 bis 5, dadurch gekennzeichnet, daß der wäßrigen Mischung lösliche Sulfate oder Phosphate zugesetzt werden und daß das nach der Gelierung erhaltene Produkt mit der Lösung von Salzen behandelt wird, deren Kationen schwerlösliche Sulfate oder Phosphate bilden.

## Claims

1. A process for the preparation of an enzymatically active formulation embedded in silica gel, wherein an aqueous mixture of an enzymatically active formulation and a dissolved alkali metal silicate and/or ammonium silicate is suspended in an organic, water-immiscible fluid and then converted into a water-insoluble gel.

2. A process as claimed in claim 1, wherein the enzymatically active formulation used consists of active cells or cell fragments of microbiological, vegetable, animal or human origin.

3. A process as claimed in claim 1, wherein the formation of a suspension is assisted by using a suspending agent.

4. A process as claimed in claims 1 to 3, wherein the gelling is effected by lowering the pH by means of an agent, preferably an organic acid, which is soluble in water and in the organic water-immiscible fluid.

5. A process as claimed in claims 1 to 4, wherein the gelling is effected in the presence of an inert material.

6. A process as claimed in claims 1 to 5, wherein the soluble sulfates or phosphates are added to the aqueous mixture and the product obtained after gelling is treated with a solution of a salt of which the cation forms a sparingly soluble sulfate or phosphate.

## Revendications

1. Procédé de préparation de produits à activité enzymatique enrobés dans un gel de silice,

7

caractérisé en ce que l'on prépare une suspension d'un mélange aqueux de produits à activité enzymatique et de silicates de métaux alcalins et (ou) d'ammonium dissous dans un liquide organique non miscible à l'eau, que l'on transforme ensuite en un gel insoluble dans l'eau.

2. Procédé suivant la revendication 1, caractérisé en ce que les produits à activité enzymatique sont des cellules ou fragments cellulaires actifs d'origine microbiologique, végétale, animale ou humaine.

3. Procédé suivant la revendication 1, caractérisé en ce que la formation de la suspension est facilitée par l'emploi d'un additif de suspension.

4. Procédé suivant l'une des revendications 1 à 3, caractérisé en ce que la gélification est provoquée par l'abaissement du pH à l'aide d'un agent chimique soluble dans l'eau et dans le liquide organique non miscible à l'eau, de préférence un acide organique.

5. Procédé suivant l'une des revendications 1 à 4, caractérisé en ce que la gélification est réalisée en présence d'une matière inerte.

6. Procédé suivant l'une des revendications 1 à 5, caractérisé en ce que l'on ajoute au mélange aqueux des sulfates ou phosphates solubles et on traite le produit gélifié obtenu avec une solution de sels, dont les cations forment des sulfates ou phosphates peu solubles.